# EUROPEAN PATENT SPECIFICATION

(11) **EP 1 131 126 B1**
(45) Date of publication and mention of the grant of the patent: **11.08.2004**
(21) Application number: 99963931.3
(22) Date of filing: 19.11.1999
(51) Int. Cl.: A61M 25/10, A61F 5/453

(54) **DOUBLE-LAYERED NON-POROUS BALLOON CATHETER**
DOPPELWANDIGER, NICHTPORÖSER BALLONKATHETER
CATHETER A BALLONNET NON POREUX DOUBLE COUCHE

(30) Priority: 19.11.1998 US 109051 P
(43) Date of publication of application: 12.09.2001
(73) Proprietor: Cook Urological Inc., Spencer, IN 47460 (US)
(72) Inventor: ROEMER, Frederick, D., Bloomington, IN 47404 (US)
(74) Representative: Johnston, Kenneth Graham
(86) International application number: PCT/US1999/027419
(87) International publication number: WO 2000/029059

(56) References cited:
- EP-A- 0 214 721
- EP-A- 0 341 988
- FR-A- 2 364 645
- FR-A- 2 639 220
- US-A- 5 358 487
- US-A- 5 409 495

## Description

This invention relates generally to medical devices, and more particularly to catheterization devices including an inflatable balloon which engages a portion of a patient. The invention is defined in independent claim 1.

Balloon catheters are well-known devices used for a variety of purposes in the medical field. Balloon catheters include an inflatable catheter balloon carried by a catheter. The inflatable balloon is filled with an inflation fluid, either a gas or a liquid, to occlude and seal a body space, or to expand a blood vessel, or to have other beneficial effects in a patient. Balloon catheters are used in angioplasty, Foley catheterization, condom catheterization, esophageal catheterization, gastric (or belly) catheterization and nasal pharyngeal catheterization, among many other procedures.

One example for a balloon catheter can be found in EP 0 214 721.)

For a variety of reasons, it is often highly desirable to employ on a balloon catheter a balloon composed or constructed of silicone. Silicone is of course a siloxane polymer, most commonly a linear or cyclic organosiloxane polymer. The following materials are included within the meaning of the word "silicone": polysiloxane, silicone rubber and Silastic silicone (product of Dow Corning). Silicone is advantageous for use as a balloon material in a balloon catheter because it is a highly biocompatible elastomer, one that seals well against tissues of the patient without causing undue irritation or the like. Silicone is also readily sterilized, has good elasticity and other desirable mechanical properties, and has an acceptable cost.

Unfortunately, the use of silicone as a material for the balloon in a balloon catheter is subject to several drawbacks. Perhaps most importantly, silicone is somewhat permeable to air, water or the other common balloon inflation media; a silicone balloon can lose a significant amount of its pressure over a relatively short time, on the order of several hours, or overnight, because of its permeability.

This drawback has made silicone a less than desirable substitute for the latex previously employed for balloons in balloon catheters. Latex is typically substantially less permeable to the common inflation media than is silicone, and would be a desirable balloon material in balloon catheters for many reasons. However, the trend appears to be to refrain from using latex as a balloon material because of the sensitivity (or even allergic reactions) some patients exhibit towards latex, and because of recently increased labeling requirements imposed by the U.S. Food and Drug Administration with regards to devices including a latex balloon.

Simple balloon catheters generally have cost and reliability advantages over catheters employing more complex structures for holding a catheter in position on or in a patient. The urinary collection or condom catheters disclosed in U.S. Patents No. 5,618,277 (Apr. 8, 1997) and No. 5,380,312 (Jan. 10, 1995), each issued to Goulter, are held on the penis by an undesirably complex arrangement which includes raised ribs and an elastic band, in addition to sequential balloons acting as a skin shield or a condom sheath. The device disclosed in U.S. Patent No. 5,662,631 (Marx, Sep. 2, 1997) employs reduced pressure applied within a rigid catheter sleeve, which applies reduced pressure to the penis to retain the device on the patient. The drawbacks from both the applied reduced pressure and the rigid sleeve should be immediately apparent, patient comfort being a significant concern in catheters intended for urinary collection.

In the context of angioplasty, it has been proposed to employ a laminated or multi-layered balloon composed of diverse polymeric compounds having different properties. The multilayer extrusion of such balloons is disclosed in U.S. Patent No. 5,270,086 (Hamlin, Dec. 14, 1993). That patent employs an adhesion or bonding layer in a balloon to promote the bonding of a high tensile strength outer layer of the balloon. The resulting structure is purported to have more desirable failure characteristics, because the materials from which the balloon is extruded are ones not prone to pinhole leaks. The desirable silicones mentioned above, of course, are indeed subject to leaks, whether from pinholes, or otherwise.

The problem of pinholes in angioplasty balloon catheters has also been addressed in U.S. Patent No. 5,478,320 (Trotta, Dec. 26, 1995), which employs a second layer to seal any pinholes present in a first layer of a balloon. The second layer is purported to solve the problem because a pinhole will only have an appreciable negative effect if all layers exhibit aligned pinholes. Such a solution would be ineffective in cases where a desired balloon material was actually porous, rather than perforate, or was so perforate under normal conditions of manufacture that there was always some degree of alignment of the pinholes present in different layers.

It would be highly advantageous to have a balloon catheter which included a balloon which exhibited the good biocompatibility properties of silicone, but which was substantially less permeable to common inflation media than silicone. It would also be highly advantageous for the balloon to be composed of a material or materials which were relatively easy to process and which were generally competitive in cost with the materials presently in use for balloons on balloon catheters. Finally, it would be highly advantageous to have a balloon catheter which lacked the rigidity or structural complexity found in prior devices, for example, in prior urinary collection catheters.

### Brief Description of the Drawings

FIG. 1 is a cross-sectional view of a first preferred embodiment of the present invention;
FIG. 2 is a cross-sectional view of the embodiment of the present invention shown in FIG. 1, during use;
FIG. 3 is a cross-sectional view of a portion of another preferred embodiment of the present invention, similar to part of FIG. 2;
FIG. 4 is a cross-sectional view of another preferred embodiment of the present invention; and
FIG. 5 is a cross-sectional view of the embodiment of the present invention shown in FIG. 4, during use.

### Detailed Description

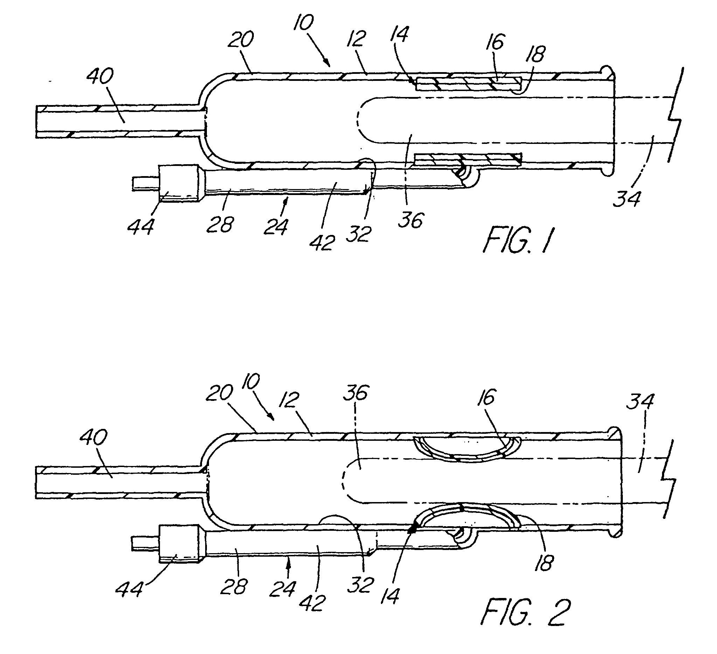

With reference first to FIGs. 1 (deflated) and 2 (inflated), a first embodiment of a balloon catheter 10 according to the present invention is thereshown, configured as a condom catheter 20 useful for the collection of urine from a male patient. The balloon catheter 10 first comprises a catheter 12 of any convenient or conventional composition. The catheter 12 should be composed of a suitable medical grade material which is impermeable to any inflation medium to the degree necessary to render the balloon catheter 10 operational. "Catheter 12" includes a complete catheter, or just a portion of a catheter of more complex structure.

The balloon catheter 10 of the present invention next comprises an inflatable catheter balloon 14 carried by the catheter 12. In the condom catheter 20, the balloon 14 is positioned on an internal surface 32 of the catheter 12. The balloon 14 is dimensioned and is arranged on the catheter 12 in a manner that, upon inflation by filling with an appropriate inflation medium or fluid (either gas or liquid), the balloon 14 expands and applies pressure against patient tissue 34 contained in the condom catheter 20. In this particular case, the patient tissue 34 is exemplified by the penis 36, and the condom catheter 20 includes an outlet 40 opposite the patient for the disposal of urine collected from the patient.

The balloon catheter 10 preferably further comprises means 24 for connecting the inflatable balloon 14 to a supply of an inflation fluid for inflating the balloon 14. In the condom catheter 20, the connecting means 24 preferably comprises a fluid connection 28 which is formed other than within the catheter 12. For example, the fluid connection 28 can comprise an exterior tube 42 in fluid communication with the interior of the balloon 14, and a fitting 44 on the tube 42 for connecting the tube 42 to a conventional supply of inflation medium.

As mentioned above, the key to the present invention is the structure of the inflatable catheter balloon 14 carried on the catheter 12 of the balloon catheter 10. More particularly, the catheter balloon 14 comprises at least an inner portion 16 and an outer portion 18 over the inner portion. "Inner" and "outer" may refer, but do not necessarily refer, to any particular position with regard to the overall structure of the balloon catheter 10 itself, nor the catheter 12. Rather, "outer" refers to that portion of the balloon 14 which will lie nearest the tissue 34 of the patient during use of the balloon catheter 10, while "inner" refers to that portion of the balloon 14 which will lie nearest the inflation gas during use of the balloon catheter 10. Preferably, the outer portion 18 is to cover all of the tissue-adjacent surfaces of the balloon.

The outer portion 18 of the balloon 14 comprises a silicone, that is, it can include, can be composed of or can consist of a silicone. The silicone is preferably a medical grade material. The present invention lies in providing an inner portion 16 of the balloon 14 which is less porous than the outer silicone portion 18 of the balloon 14. The result is that leakage of the inflation medium through the catheter balloon 14 when inflated, and in particular, through the inner portion of the balloon 14, is less than the leakage which would occur through a hypothetical, similarly dimensioned silicone catheter balloon, when comparably inflated.

The inner and outer portions 16 and 18 of the catheter balloon 14 can be, but need not necessarily be, connected along their entire surfaces. The inner and outer portions 16 and 18 can be chemically bonded to one another, and this may be of particular commercial importance, since silicones can often be polymerized in place upon a suitable substrate, the inner portion 16 serving as such a substrate. Alternatively, either or both of the inner and outer portions 16 and 18 of the inflatable balloon 14 can comprise discrete balloons, connected along their surfaces or not. For example, it may be preferred that the inner portion 16 of the balloon 14 comprise a discrete balloon, and the outer portion 18 of the balloon comprise a layer on the inner portion 16 of the balloon 14. The outer portion 18 could, in such a case, be a silicone which is polymerized on the discrete balloon making up the inner portion 16. The outer portion 18 could instead comprise a discrete balloon, and the inner portion 16 formed as a layer on the discrete balloon making up the outer portion 18 of the balloon 14. Such discrete balloons are not specifically shown in the drawing Figures, however, since in both the deflated (FIG. 1) and inflated (FIG. 2) conditions of the balloon catheter 10, the appearance of the inner and outer portions 16 and 18 of the balloon 14 would appear generally the same, no matter how they are layered.

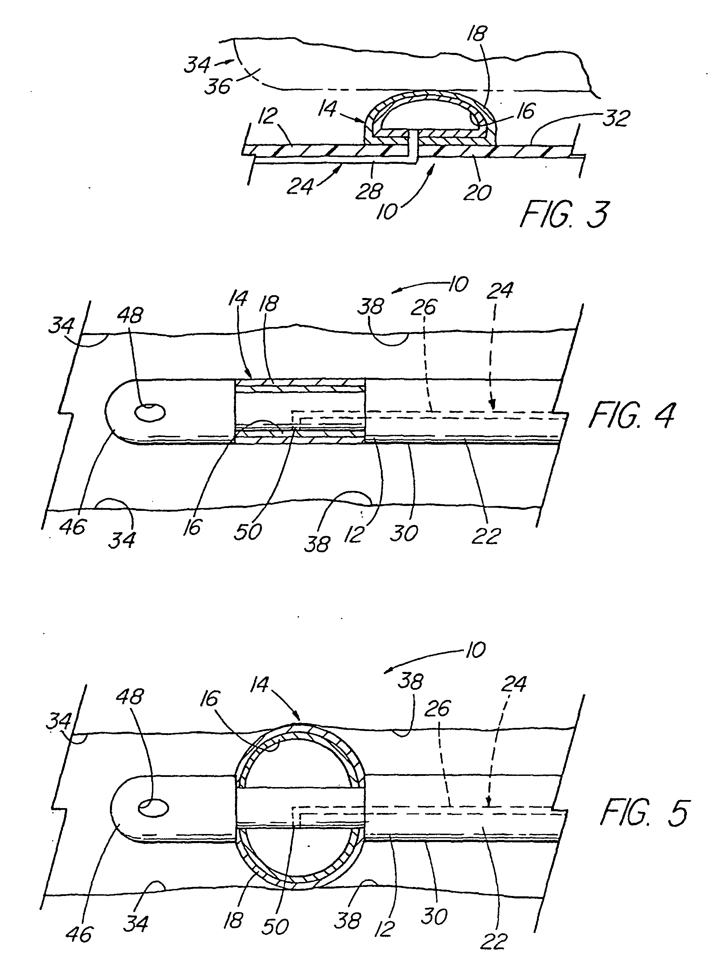

It may be preferred in some cases that the outer portion 18 of the catheter balloon 14 completely encapsulate or surround the inner portion 16 of the balloon 14. A detail of this arrangement, similar to part of the view of FIG. 2, is shown in FIG. 3. In such an arrangement, it may not even be necessary to affix the inflatable balloon 14 to the catheter 12 of the balloon catheter 10 at all; the inflation pressure of the balloon 14 may be sufficient to hold the catheter 12 in place with respect to the tissue 34 of the patient.

Of course, the balloon catheter 10 need not be configured just as a condom catheter 20. As shown in FIGs. 4 (deflated condition) and 5 (inflated condition), the balloon catheter 10 can instead be configured as a Foley catheter 22. The Foley catheter 22 is an example of other suitable configurations for the balloon catheter 10, such as an esophageal catheter, a gastric balloon catheter, a nasal pharyngeal balloon catheter, or the like. For brevity, only the Foley catheter 22 will be described in detail, since those skilled in the art should readily and immediately appreciate any minor changes in arrangement, composition or construction needed to adapt a Foley catheter to those particular uses.

In the Foley catheter 22, the inflatable balloon catheter 14 is positioned on an external surface 30 of the catheter 12, between the catheter 12 and patient tissue 34 (exemplified by a body passage 38) which surrounds the Foley catheter 22. The means 24 for connecting the inflatable balloon 14 to the supply of inflation medium or fluid comprises a conventional conduit, passage, lumen or bore 26 formed in the catheter 12, fluidly connected to the interior of the inflatable balloon 14. The connection of the conduit or the like 26 to the source of the medium or fluid is not shown in FIGs. 4 and 5, but fluid communication of the conduit or the like 26 with the interior of the balloon 14 is provided by an opening 50 formed in the catheter 12. In the conventional manner, the distal end 46 of the Foley catheter 22 is provided with an operative opening 48, in communication with a lumen formed in the catheter 12 (not shown) for conventional purposes.

In any of these embodiments of the invention, the inner portion 16 of the inflatable catheter balloon 14 can comprise, include, be composed of or consist of any of a variety of materials. Preferably, the inner portion 16 of the balloon 14 comprises isoprene, neoprene or Buna-N (nitrile). Most preferably, the inner portion 16 of the balloon 14 comprises latex. However, many other medical grade elastomers are also expected to be useful in the practice of the present invention. Thus, the inner portion 16 of the balloon 14 may alternatively comprise butadiene-styrene copolymer, polyisobutylene, polyurethane, a fluoroelastomer such as Viton®, a chlorosulfonated polyethylene such as Hypalon®, SBR (styrene-butadiene rubber), EPDM (a terpolymer elastomer made from ethylene-propylene diene monomer) or PVC (polyvinyl chloride). Viton® is believed to be a trademark of Du Pont (Wilmington, Delaware, USA) for a series of fluoroelastomers based on the copolymer of vinylidine fluoride and hexafluoropropylene. Hypalon® is believed to be a trademark of Du Pont for chlorosulfonated polyethylene, a synthetic rubber. Other medical grade elastomers may be useful as well, so long as they are less porous to conventional balloon inflation media or fluids than is the silicone making up the outer portion 18 of the catheter balloon 14.

The inflatable balloon 14 of the balloon catheter 10 of the present invention may of course include more layers or portions than the inner portion 16 and the outer portion 18 disclosed above. The particular circumstances of use may suggest whether additional layers or portions would be useful. Without regard to the presence of additional layers or portions, a balloon catheter 10 constructed in accordance with the present invention would be used in the same way as a balloon catheter possessing a conventional inflatable balloon. Those skilled in the art are well aware of the details of such use, and for brevity, those details will be omitted here. Moreover, non-distending balloons 14 are certainly contemplated within the scope of the present invention.

It should be clear that the present invention provides a balloon catheter 10 which possesses significant advantages over prior balloon catheters. The balloon 14 of the balloon catheter 10 of the present invention exhibits the good biocompatibility properties of silicone, but is substantially less permeable to common inflation media than a silicone balloon would be. The balloon catheter 10 of the present invention is relatively easy to manufacture and is generally competitive in cost with balloon catheters employing balloons which do not enjoy the advantages enjoyed by its own balloon 14. Finally, the balloon catheter 10 of the present invention should be relatively comfortable to patients during use, because it lacks the rigidity and structural complexity found in prior devices, for example, in prior urinary collection catheters.

The details of the construction or composition of the various elements of the balloon catheter 10 of the present invention not otherwise disclosed are not believed to be critical to the achievement of the advantages of the present invention, so long as the elements possess the strength or mechanical properties needed for them to perform as disclosed. The selection of any such details of construction are believed to be well within the ability of one of even rudimentary skills in this area, in view of the present disclosure.

The present invention is useful for catheterization of a patient, and therefore finds applicability in human and veterinary medicine.

It is to be understood, however, that the above-described balloon catheter is merely an illustrative embodiment of this invention, and that other devices and methods for using them may be devised by those skilled in the art. It is also to be understood that the invention is directed to embodiments both comprising and consisting of the disclosed parts.

## Claims

1. A balloon catheter (10) comprising a catheter (12) and an inflatable catheter balloon (14) carried by the catheter (12); the catheter balloon (14) comprising at least an inner portion (16) and an outer portion (18) over the outer surface of the inner portion to be adjacent to the tissue surfaces of a patient; the outer portion (18) comprising a silicone, and the inner portion (16) being less porous than the outer portion (18); **characterized in that** the outer portion completely encapsulates the entire inner portion and extends between the inner portion and the catheter.

2. A balloon catheter according to claim 1, wherein the inner and outer portions are chemically bonded to one another.

3. A balloon catheter according to claim 2 wherein the inner and outer portions are bonded to one another by polymerization.

4. A balloon catheter according to claim 1 wherein the outer portion is in the form of a discrete balloon.

5. A balloon catheter according to claim 1 wherein the balloon catheter (10) is configured as a Foley catheter (22).

6. A balloon catheter (10) according to claim 1, wherein the balloon catheter (10) is configured as an esophageal catheter, a gastric balloon catheter or a nasal pharyngeal balloon catheter.

7. The balloon catheter (10) according to claim 1, further comprising means (24) for connecting the inflatable balloon (14) to a supply of an inflation fluid for inflating the balloon (14).

8. The balloon catheter (10, 22) according to claim 7, wherein the connecting means (24) comprises a conduit, passage, lumen or bore (26) formed in the catheter (12), fluidly connected to the inflatable balloon (14).

9. The balloon catheter (10, 20) according to claim 7, wherein the connecting means (24) comprises a fluid connection (28) which does not completely pass through the catheter (12).

10. The balloon catheter (10) according to claim 1, wherein the inner portion (16) of the inflatable balloon (14) comprises latex, isoprene, neoprene or Buna-N (nitrile).

11. The balloon catheter (10) according to claim 1, wherein the inner portion (16) of the inflatable balloon (14) is elastomeric.

12. The balloon catheter (10) according to claim 11, wherein the inner portion (16) of the inflatable balloon (14) comprises butadiene-styrene copolymer, polyisobutylene, polyurethane, a fluoroelastomer, a chlorosulfonated polyethylene, SBR, EPDM or PVC.

## Patentansprüche

1. Ballonkatheter (10), umfassend einen Katheter (12) und einen aufblasbaren Katheterballon (14), der vom Katheter (12) getragen wird, wobei der Katheterballon (14) zumindest einen inneren Abschnitt (16) und einen äußeren Abschnitt (18) über der Außenseite des inneren Abschnitts, der an den Gewebsflächen eines Patienten anliegen soll, umfasst, wobei der äußere Abschnitt (18) aus einem Silikon besteht und der innere Abschnitt (16) weniger porös ist als der äußere Abschnitt (18), **dadurch gekennzeichnet, dass** der äußere Abschnitt den gesamten inneren Abschnitt vollständig umschließt und zwischen dem inneren Abschnitt und dem Katheter verläuft.

2. Ballonkatheter nach Anspruch 1, bei dem der innere und der äußere Abschnitt chemisch miteinander verbunden sind.

3. Ballonkatheter nach Anspruch 2, bei dem der innere und der äußere Abschnitt durch Polymerisation miteinander verbunden sind.

4. Ballonkatheter nach Anspruch 1, bei dem der äußere Abschnitt die Form eines diskreten Ballons aufweist.

5. Ballonkatheter nach Anspruch 1, bei dem der Ballonkatheter (10) als Foley-Katheter (22) ausgebildet ist.

6. Ballonkatheter (10) nach Anspruch 1, bei dem der Ballonkatheter (10) als Ösophagus-Katheter, Magen-Ballonkatheter oder Nasen/Pharynx-Ballonkatheter ausgebildet ist.

7. Ballonkatheter (10) nach Anspruch 1, ferner umfassend ein Mittel (24) zum Verbinden des aufblasbaren Ballons (14) mit einer Inflationsfluid-Quelle zum Aufblasen des Ballons (14).

8. Ballonkatheter (10, 22) nach Anspruch 7, bei dem das Verbindungsmittel (24) eine Leitung, einen Durchgang, ein Lumen oder eine Bohrung (26) umfasst, die/der/das im Katheter (12) geformt ist und in Fluidverbindung mit dem aufblasbaren Ballon (14) steht.

9. Ballonkatheter (10, 20) nach Anspruch 7, bei dem das Verbindungsmittel (24) eine Fluidverbindung (28) enthält, die nicht vollständig durch den Katheter (12) verläuft.

10. Ballonkatheter (10) nach Anspruch 1, bei dem der innere Abschnitt (16) des aufblasbaren Ballons (14) aus Latex, Isopren, Neopren oder Buna-N (Nitril) besteht.

11. Ballonkatheter (10) nach Anspruch 1, bei dem der innere Abschnitt (16) des aufblasbaren Ballons (14) elastomer ist.

12. Ballonkatheter (10) nach Anspruch 1, bei dem der innere Abschnitt (16) des aufblasbaren Ballons (14) aus Butadien-Styrol-Copolymer, Polyisobutylen, Polyurethan, einem Fluorelastomer, einem chlorsulfonierten Polyethylen, SBR, EPDM oder PVC besteht.

## Revendications

1. Cathéter à ballonnet (10) comprenant un cathéter (12) et un ballonnet de cathéter gonflable (14) monté sur le cathéter (12) ; le ballonnet de cathéter (14) comprenant au moins une partie intérieure (16) et une partie extérieure (18) située sur la surface extérieure de la partie intérieure à positionner de façon adjacente aux surfaces des tissus d'un patient ; la partie extérieure (18) comprenant une silicone, et la partie intérieure (16) étant moins poreuse que la partie extérieure (18) ; **caractérisé en ce que** la partie extérieure englobe entièrement toute la partie intérieure et s'étend entre la partie intérieure et le cathéter.

2. Cathéter à ballonnet selon la revendication 1, dans lequel les parties interne et externe sont chimiquement fixées l'une à l'autre.

3. Cathéter à ballonnet selon la revendication 2, dans lequel les parties interne et externe sont fixées l'une à l'autre par polymérisation.

4. Cathéter à ballonnet selon la revendication 1, dans lequel la partie externe se présente sous la forme d'un ballonnet discret.

5. Cathéter à ballonnet selon la revendication 1, dans lequel le cathéter à ballonnet (10) est configuré comme une sonde de Foley (22).

6. Cathéter à ballonnet (10) selon la revendication 1, dans lequel le cathéter à ballonnet (10) est configuré comme une sonde oesophagienne, une sonde gastrique à ballonnet ou une sonde nasopharyngienne à ballonnet.

7. Cathéter à ballonnet (10) selon la revendication 1, comprenant en outre un moyen (24) de raccordement du ballonnet gonflable (14) à une alimentation d'un fluide de gonflement pour le gonflement du ballonnet (14).

8. Cathéter à ballonnet (10, 22) selon la revendication 7, dans lequel le moyen de raccordement (24) comprend un conduit, un passage, une lumière ou un alésage (26) formé(e) dans le cathéter (12), raccordé(e) de façon fluide au ballonnet gonflable (14).

9. Cathéter à ballonnet (10, 20) selon la revendication 7, dans lequel le moyen de raccordement (24) comprend un raccord de fluide (28) qui ne traverse pas complètement le cathéter (12).

10. Cathéter à ballonnet (10) selon la revendication 1, dans lequel la partie intérieure (16) du ballonnet gonflable (14) comprend du latex, de l'isoprène, du néoprène ou du Buna-N (nitrile).

11. Cathéter à ballonnet (10) selon la revendication 1, dans lequel la partie intérieure (16) du ballonnet gonflable (14) est en élastomère.

12. Cathéter à ballonnet (10) selon la revendication 11, dans lequel la partie intérieure (16) du ballonnet gonflable (14) comprend un copolymère butadiène-styrène, du polyisobutylène, du polyuréthane, un fluoroélastomère, du polyéthylène chlorosulfoné, du SBR, un EPDM ou du PVC.
